# EUROPÄISCHE PATENTSCHRIFT

(11) **EP 2 259 829 B1**
(45) Veröffentlichungstag und Bekanntmachung des Hinweises auf die Patenterteilung: **20.11.2013**
(21) Anmeldenummer: 09712727.8
(22) Anmeldetag: 20.02.2009
(51) Int. Cl.: A61M 16/20, A61M 16/06

(54) **APPLIKATOREN FÜR EINE LUFTBRILLE**
APPLICATORS FOR A NASAL CANNULA
APPLICATEURS POUR LUNETTE À AIR

(30) Priorität: 21.02.2008 DE 102008010475
(43) Veröffentlichungstag der Anmeldung: 15.12.2010
(73) Patentinhaber: TNI medical AG, 97084 Würzburg (DE)
(72) Erfinder: GENGER, Harald, 74906 Bad Rappenau (DE); WALTHER, Heide, 07745 Jena (DE)
(74) Vertreter: advotec.
(86) Internationale Anmeldenummer: PCT/DE2009/050008
(87) Internationale Veröffentlichungsnummer: WO 2009/103288

(56) Entgegenhaltungen:
- EP-A- 1 317 941
- WO-A-2006/072231
- WO-A-2008/014543
- US-A- 4 782 832
- US-A1- 2005 028 823

## Beschreibung

Das Gebiet der Erfindung sind Applikatoren für Luftbrillen der im Oberbegriff des Patentanspruchs 1 genannten Art. Solche Applikatoren sind aus der US 2005/0028823 A1 bekannt.

Bei der CPAP-Therapie (Continous Positive Airway Pressure-Therapie) wird einem Patienten ein konstanter Überdruck gegenüber dem Umgebungsluftdruck über eine Nasenmaske zugeführt. Bei richtiger Wahl des Überdrucks gewährleistet dieser, dass die oberen Atemwege während der gesamten Nacht vollständig geöffnet bleiben und somit keine obstruktiven Atemstörungen auftreten. Man spricht auch von pneumatischer Schienung der Atemwege. Der erforderliche Überdruck hängt unter anderem von dem Schlafstadium und der Körperposition des Schlafenden ab. Um den als unangenehm empfundenen Überdruck auf das erforderliche Maß zu beschränken, offenbart WO 02/083221 A2 ein Therapiegerät (AutoCPAP), das den Überdruck automatisch einstellt und damit an das Schlafstadium und die Körperposition anpasst.

Um das Atmen zu erleichtern, wurden ferner BiPAP-Geräte und Multilevel-Geräte entwickelt. Diese Geräte haben die Eigenschaft, den Patienten beim Atmen dadurch zu unterstützen, dass beim Ausatmen der Überdruck abgesenkt und beim Einatmen der Überdruck wieder erhöht wird. Diese Geräte arbeiten also mit mindestens zwei Druckniveaux. Solche Geräte sind beispielsweise aus der DE 691 32 030 T2 und der WO 02/26283 A2 bekannt.

Ferner sind aus dem Stand der Technik Sauerstoffbrillen für die Sauerstoffbehandlung bekannt. Mit der Sauerstoffbrille wird dem Patienten Luft mit einem erhöhten Sauerstoffpartialdruck (> 210 mbar) oder reiner Sauerstoff in die Nase appliziert. Eine Sauerstoffbehandlung findet zum Beispiel bei akuter oder chronischer Hypoxämie infolge Atem- oder Herzkreislaufstörung (Myokardinfarkt, Schock) oder bestimmten Vergiftungen, zum Beispiel durch Kohlenmonoxid, Kohlendioxid, Leuchtgas oder Rauch statt.

Aus der WO 02/062413 A2 ist der Einsatz von Sauerstoffbrillen in einem Antischnarchgerät bekannt. Sauerstoffbrillen werden in diesem Zusammenhang als Luftbrillen bezeichnet. Aus der WO 02/062413 A2 sind ferner Luftbrillen mit integrierten Strahlpumpen bekannt, wie sie in Fig. 4 und 5 der WO 02/062413 A2 dargestellt sind.

Die US 2003/0079749 A1 und die WO 2006/072231 A2 beschreiben Luftbrillen, bei deren Nasenstücken Kanten abgerundet sind, an denen die Luft vorbei streicht. So werden Rausch- und Pfeifgeräusche weit gehend vermieden.

Die FR 2 827 778 offenbart eine Vorrichtung, die als monolithisches Teil bezeichnet wird und dem Nasenteil einer Luftbrille ähnelt. Die Vorrichtung dient dazu, die Atmung eines Patienten ohne oder mit unzureichender Spontanatmung über die Nasenlöcher zu unterstützen. Die Abmessungen sind an Frühgeborene angepasst. Distale, röhrenförmige Elemente ragen in die Nasenlöcher. Schaumscheiben um die röhrenförmigen Elemente dienen als nachgiebiger Anschlag. In einer anderen Ausführungsformen stecken die röhrenförmigen Elemente in zwei domförmigen Muffen, die an der der Nase abgewandten Seite der Muffen über eine Brücke verbunden sind. Zwei Leitungen werden parallel mit einem atembaren Gas versorgt. Ein Kappillarschlauch dient als Drucksonde. Am Beginn einer Inspirationsphase empfängt eine Versorgungseinrichtung über den Kappillarschlauch einen Druckabfall und kann dem Patienten einen kontinuierlichen oder impulsförmigen Strahl atembares Gas zuführen. Nach einer Inspirationsphase wird die Versorgungseinrichtung durch den über den Kapillarschlauch übertragenen Druck angewiesen, die Gaszufuhr einzustellen. So kann der Patient frei ausatmen.

Die US 2005/0028823 A1 offenbart einen Applikator zur nasalen Beatmung. Der Applikator hat einen hohlen Körper mit zwei Nasenöffnungen und zwei Einatmungsöffnungen. An jeder Einatmungsöffnung befindet sich ein Schlauchverbinder, der über ein Schlauchsystem mit einer Gasversorgung, beispielsweise einem Sauerstofftank verbunden ist. In einer zweiten Ausführungsform weist der hohle Körper eine oder zwei Ausatmungsöffnungen und eine oder mehrere Ventilbaugruppen auf. Jede Ventilbaugruppe kann zwei Ein-Weg-Einatmungsventilmembranen und zwei Ein-Weg-Ausatmungsventilmembranen aufweisen. Die Einatmungsventilmembranen können im Körper zwischen einer der Nasenöffnungen und einer der Ausatmungsöffnungen oder innerhalb der Einatmungsöffnungen angeordnet sein. Die Ausatmungsventilmembranen können innerhalb der Ausatmungsöffnungen angebracht sein.

Es ist Aufgabe der Erfindung, Applikatoren für Luftbrillen anzugeben, die den Atemkomfort des Patienten erhöhen.

Diese Aufgabe wird durch die Lehre des unabhängigen Anspruchs gelöst.

Bevorzugte Ausführungsformen der Erfindung sind Gegenstand der Unteransprüche.

Überraschend vorteilhaft an einem Ventil in einer Wand des Körpers des Applikators, wobei das Ventil Gas nur aus der Umgebung in den Körper des Applikators strömen lässt, ist, dass bei einem Defekt des angeschlossenen Verdichters für den Benutzer keine Erstickungsgefahr besteht.

Durch einen Aufsatz, der auf die Zinken aufgesteckt ist, kann die Außenform der Zinken in überraschend einfacher Weise an die Innenform der Nasenlöcher eines Patienten angepasst werden. Somit weist lediglich der kleine, relativ einfach geformte Aufsatz eine patientenspezifische Form auf, nicht aber die große, relativ kompliziert geformte Luftbrille. Somit werden für die Luftbrille größere Stückzahlen erreicht, was die Kosten senkt.

Eine Brücke zwischen den beiden Dichtkegeln des Aufsatzes auf ihrer, dem Körper (11) zugewandten Seite verhindert, dass ein einzelner Dichtkegel verloren geht. Darüberhinaus wird in überraschend einfacher Weise ein Verdrehen der Dichtkegel auf den Zinken unterbunden. Schließlich verhindert die Brücke auch ein unabsichtliches Abziehen des Aufsatzes von den Zinken.

Kranzförmige Dichtlippen auf der dem Körper des Applikators abgewandten Seite der Dichtkegel stellen eine angenehme, dichte Verbindung mit der Innenwand des entsprechenden Nasenlochs her.

Weil die Dichtkegel zwischen einem inneren Teil und einem äußeren Kegel teilweise hohl sind, so dass zwischen dem inneren Teil (31, 32) und dem entsprechenden äußeren Kegel (33, 34) Gas parallel zum inneren Teil (31,32) fließen kann, wird der zusäzliche Luftwiderstand im Nasenloch durch den Applikator gering gehalten.

Vorteilhaft an den membranbelasteten Auslässen am körperseitigen Ende der äußeren Kegel ist, dass der Benutzer ausgeatmete, verbrauchte Luft nicht wieder einatmet. Darüberhinaus kann durch die Wahl der Festigkeit der beilagscheibenförmigen Membranen der durch den Applikator erzeugte Überdruck eingestellt werden, was die Steuerung des angeschlossenen Verdichters vereinfacht.

Der Wulst am nasenseitigen Ende der Zinken verhindert in vorteilhafter Weise ein unbeabsichtigtes abziehen des Aufsatzes.

Die Blende ermöglicht eine einfache Montage der flexiblen Membran.

Im Folgenden wird eine bevorzugte Ausführungsform der Erfindung unter Bezugnahme auf die beiliegenden Zeichnungen näher erläutert. Dabei zeigen:
Fig. 1 eine Vorderansicht eines erfindungsgemäßen Applikators;
Fig. 2 eine Draufsicht auf einen erfindungsgemäßen Applikator;
Fig. 3 eine Rückansicht eines erfindungsgemäßen Applikators;
Fig. 4 eine Untersicht eines erfindungsgemäßen Applikators;
Fig. 5 die Vorderansicht aus Fig. 1 ohne Aufsatz und Blende;
Fig. 6 die Untersicht aus Fig. 4 ohne Aufsatz und Blende; und
Fig. 7 die Untersicht aus Fig. 6 ohne Membranen.

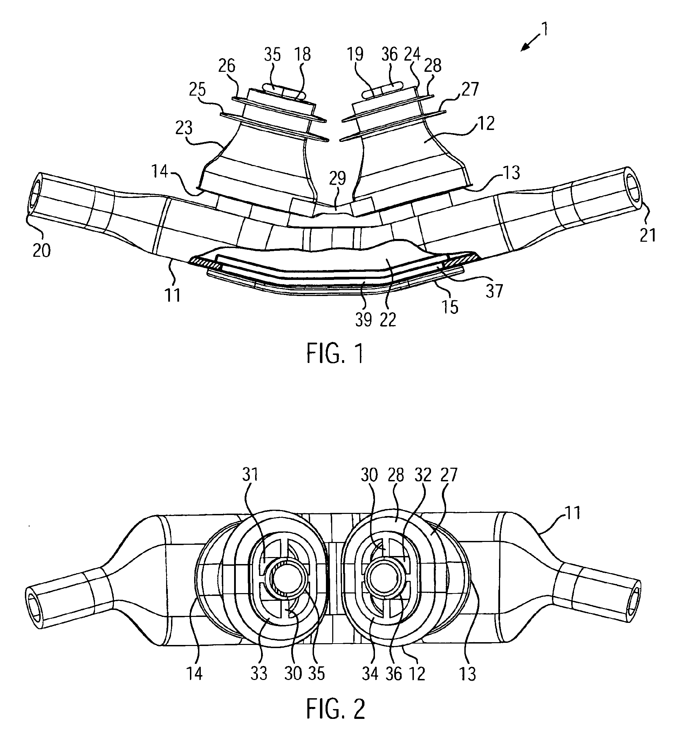

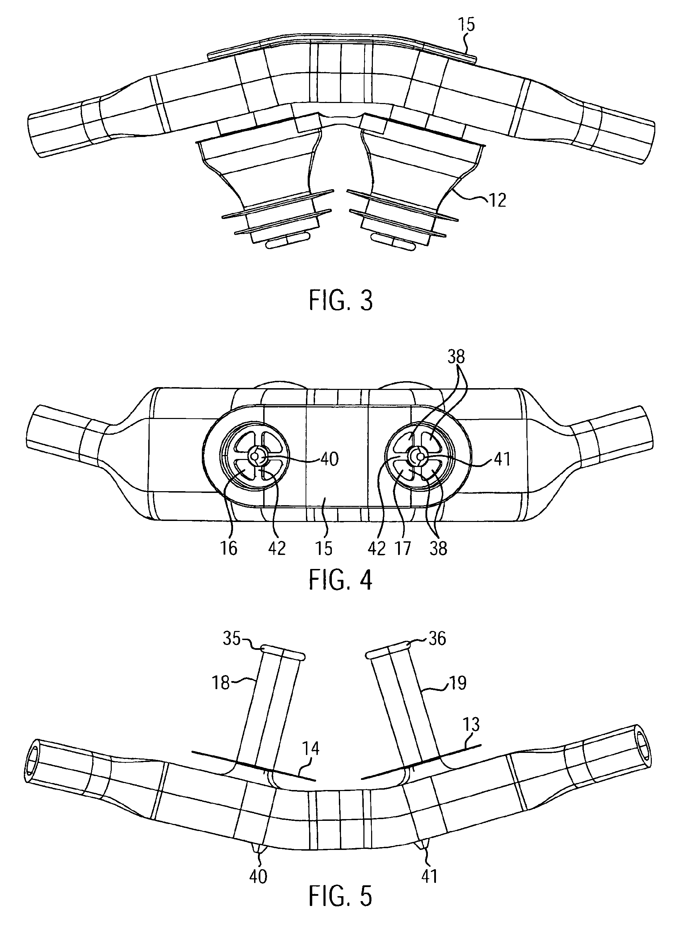

Figur 1 zeigt eine Vorderansicht des erfindungsgemäßen Applikators 1. Der Applikator 1 besteht hauptsächlich aus einem Körper 11, der über zwei Schlauchanschlüsse 20, 21 über eine nicht dargestellte Schlauchschleife an einen Verdichter angeschlossen ist. Der Körper 11 weist zwei Zinken 18, 19 auf, über die ein Aufsatz 12 gesteckt ist, der im wesentlichen aus zwei Dichtkegeln 23, 24 besteht. Synonym oder äquivalent zu einem Verdichter sind ein Kompressor, eine Pumpe, eine Druckluftquelle, eine Versorgungseinrichtung oder ein Gebläse. In Figur 1 sind im wesentlichen lediglich die Wülste 35, 36 der Zinken 18, 19 zu sehen. Die Zinken 18, 19 sind in Figur 5 ohne die Dichtkegel 23, 24 dargestellt.

Die Dichtkegel sind in Figur 1 oben, also an ihrem nasenseitigen, körperfernen Ende an die Innenform der Nasenlöcher eines Benutzers angepasst. Tatsächlich sind die Dichtkegel etwas kleiner als die Öffnungen der Nasenlöcher des Benutzers. Dieser kleine Spalt wird durch Dichtlippen 25, 26, 27 und 28 überbrückt und abgedichtet. Die Dichtlippen 25, 26, 27 und 28 selbst haben näherungsweise die Form einer Mantelfläche eines sehr flachen Kegels. Sie verlaufen also von innen oben nach außen unten. Aufgrund dieser Formgebung lassen sich die Dichtkegel leicht in die Nase einführen und stellen einen leichten Widerstand gegen Herausrutschen des Applikators aus der Nase dar.

Die Dichtkegel 23, 24 sind an ihrem unteren, nasenfernen, körpernahen Ende durch eine Brücke 29 miteinander verbunden, um ein zu leichtes Abziehen der Dichtkegel 23, 24 von den Zinken 18, 19 und ein Verdrehen der Dichtkegel 23, 24 gegenüber dem Körper 11 und den Nasenlöchern eines Benutzers zu verhindern.

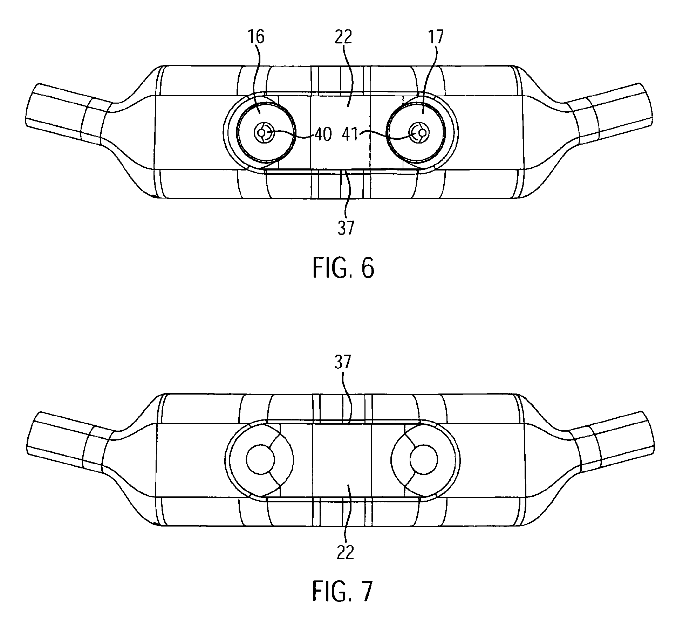

Auf der den Zinken 18, 19 gegenüberliegenden Seite des Körpers 11 weist der Körper 11 ein Langloch 37 auf, das auch in den Figuren 6 und 7 gut zu erkennen ist. Das Langloch 37 ist durch eine Blende 15 verschlossen, die zusammen mit den Membranen 16, 17 (siehe Figur 4) zwei Ventile bildet. Die Blende weist an ihrem äußeren Rand eine umlaufende Nut 39 auf, die in die Wand des Körpers 11 eingreift, die das Langloch 37 begrenzt. So klemmt sich die Blende 15 an der Wand des Körpers 11 fest und verschließt das Langloch 37 gasdicht.

Der Körper 11 umschließt einen Hohlraum 22. Insbesondere die Innenflächen des Körpers 11 weisen keine scharfen Kanten auf. Vielmehr sind alle Kanten abgerundet, um Strömungsgeräusche zu minimieren. Da die Wand des Körpers 11 in etwa die gleiche Dicke aufweist, erkennt man diese Verrundungen auch außen am Körper 11.

Die Figur 2 zeigt eine Draufsicht auf einen erfindungsgemäßen Applikator. Man erkennt, dass die Dichtkegel 23, 24 aus äußeren Kegeln 33 bzw. 34, inneren Zylindern 31 bzw. 32 sowie Rippen 30 bestehen, die die äußeren Kegel 33, 34 mit den inneren Zylindern 31, 32 mechanisch verbinden. Die Außenflächen der Zinken 18, 19 sind etwa zylinderförmig und bilden mit den Innenflächen der inneren Zylinder 31, 32 eine Passung.

Im Betrieb strömt also von einem Verdichter komprimiertes, atembares Gas von den Schlauchanschlüssen 20, 21 durch den Hohlraum 22 durch die Zinken 18, 19 in die Nase eines Benutzers. Umgekehrt strömt ausgeatmete Luft durch die Dichtkegel 23, 24, also zwischen den inneren Zylindern 31, 32 und den äußeren Kegeln 33, 34 vorbei an den Rippen 30, durch leicht nach unten gebogene Membranen 13 und 14 ins Freie. Der Benutzer atmet also praktisch keine ausgeatmete Luft wieder ein.

Wie in Figur 1 dargestellt ist, enden die äußeren Kegel 33, 34 etwas oberhalb vom Körper 11, wohingegen die inneren Zylinder 31, 32 bis an den Körper 11 heranreichen. Die Öffnungen zwischen den inneren Zylindern 31, 32 sowie den äußeren Kegeln 33, 34 sind von den Membranen 13, 14 verschlossen. Herrscht allerdings wie beim Ausatmen ein gewisser Überdruck zwischen den inneren Zylindern 31, 32 sowie den äußeren Kegeln 33, 34, werden die Membranen 13, 14 leicht nach unten gebogen, so das ausgeatmete Luft ins Freie entweichen kann. Über die Härte der Membranen 13, 14 sowie der Größe der Membranen 13, 14 zusammen mit der Größe der körperseitigen Auslässe der Dichtkugel 23, 24 kann der applizierte Überdruck eingestellt werden. Die inneren Zylinder 31, 32 weisen zumindest eine Stufe auf, die die Membranen 13, 14 gegen die äußeren Kegel 33, 34 drückt. In einer anderen Ausführungsform können die inneren Zylinder 31, 32 auch Nuten aufweisen, die die vertikalen Positionen der Membranen gegenüber den äußeren Kegeln 33, 34 fixieren.

Die Figur 3 zeigt eine Rückansicht eines erfindungsgemäßen Applikators.

Die Figur 4 zeigt eine Untersicht eines erfindungsgemäßen Applikators. In dieser Ansicht erkennt man die beiden Ventile in der Blende 15. Die Blende weist zwei runde Vertiefungen auf, die durch je ein Kreuz teilweise verschlossen sind. Zwischen den Streben 42 des Kreuzes verbleiben je vier etwa viertelkreisförmige Öffnungen 38 in der Blende 15. Die Öffnungen 38 werden von innen her durch die flexiblen Membranen 16, 17 verschlossen, so dass Luft von außen in den Hohlraum 21 treten kann, nicht aber von innen aus dem Hohlraum 21 durch die Öffnungen 38 nach außen entweichen kann. Figur 6 zeigt die gleiche Ansicht wie Figur 4, wobei jedoch die Blende 15 nicht dargestellt ist, so dass die Membranen 16, 17 in der Luft zu schweben scheinen. Die beiden Ventile ermöglichen einem Benutzer das Einatmen, auch wenn bei einem Ausfall des Kompressors kein atembares Gas über die Schlauchanschlüsse 20, 21 geliefert wird.

Die beiden Membranen 16, 17 weisen in der Mitte je einen kegelstumpfförmigen Fortsatz 40 bzw. 41 auf, der in den Figuren 4 sowie 6 dem Betrachter entgegen ragt. Die kegelstumpfförmigen Fortsätze sind über zylinderförmige Abschnitte mit den eigentlichen Membranen verbunden. Die zylinderförmigen Abschnitte haben einen geringeren Durchmesser als die größten Durchmesser der kegelstumpfförmigen Fortsätze, so dass zwischen jedem kegelstumpfförmigen Fortsatz und der entsprechenden Membran eine Nut entsteht. Diese Nut ruht in einem zentralen Loch in den Streben 42 der Kreuze in den Vertiefungen der Blende 15.

Die Figur 5 zeigt eine ähnliche Ansicht wie die Figur 1, wobei jedoch der Aufsatz 12 mit den Dichtkegeln 23, 24 sowie die Blende 15 nicht dargestellt sind. Man erkennt also die zylinderförmigen Zinken 18, 19, die Wülste 35, 36 sowie die Membranen 13, 14 besser. Auf der Unterseite ragen die kegelstumpfförmigen Fortsätze 40, 41 der Membranen 16, 17 aus dem Langloch 37 heraus.

Die Figur 7 zeigt eine ähnliche Ansicht wie die Figur 6, wobei jedoch auch noch die Membranen 16 und 17 ausgeblendet sind. In dieser Ansicht gibt das Langloch 37 durch den Hohlraum 22 den Blick frei auf die Innenseite der verrundeten Übergänge zwischen den Zinken 18, 19 und dem restlichen Körper 11. Auf der Innenseite ist der Radius der Verrundung etwa so groß wie der Innendurchmesser der Zinken 18, 19. Aus Figur 5 erkennt man, dass auf der Außenseite der Radius dieser Verrundung etwa halb so groß wie auf der Innenseite ist. Dies liegt daran, dass die Wandstärke im Bereich der Zinken 18, 19 etwa halb so groß wie im Rest des Körpers 11 ist.

In den Figuren 2, 4, 6 und 7 erkennt man, dass der Körper wesentlich breiter als die Schlauchanschlüsse 20, 21 ist. Neben dem Zweck, Platz für die Blende 15 und die darin befindlichen Ventile zu schaffen, hat diese Maßnahme auch den Zweck, die Strömungsgeschwindigkeit des Gases im Übergangsbereich zwischen den Zinken 18, 19 und dem Körper 11 durch eine Vergrößerung des Querschnitts abzusenken und somit Strömungsgeräusche zu reduzieren.

### Bezugszeichenliste

- 1: Applikator
- 11: Körper
- 12: Aufsatz
- 13, 14: Membran
- 15: Blende
- 16, 17: Membran
- 18, 19: Zinke
- 20, 21: Schlauchanschluss
- 22: Hohlraum
- 23, 24: Dichtkegel
- 25, 26, 27, 28: Dichtlippe
- 29: Brücke
- 30: Rippe
- 31, 32: innerer Zylinder
- 33, 34: äußerer Kegel
- 35, 36: Wulst
- 37: Langloch
- 38: Öffnung
- 39: Nut
- 40, 41: kegelstumpfförmiger Fortsatz
- 42: Streben

## Patentansprüche

1. Applikator für eine Luftbrille mit:
einem Körper (11), der einen Hohlraum (22) umgibt;
einem ersten Schlauchanschluss (20) und einem zweiten Schlauchanschluss (21) zum Zuführen eines atembaren Gases in den Hohlraum (22), wobei der erste und zweite Schlauchanschluss an den Körper (11) angeformt sind;
einem ersten Zinken (18) und einem zweiten Zinken (19) zur Applikation des atembaren Gases in die Nasenlöcher einer Person, wobei der erste und zweite Zinken (18, 19) an den Körper (11) angeformt sind;
ein Ventil (15,16, 17) in einer Wand des Körpers (11),
**dadurch gekennzeichnet, dass**
das Ventil so aufgebaut ist, dass atembares Gas von außen durch das Ventil (15, 16, 17, 38, 42) in den Hohlraum (22) und weiter zu den Zinken (18, 19) strömen kann, und dass andererseits ein nennenswerter Gasfluss vom Hohlraum (22) durch das Ventil (15, 16, 17, 38, 42) nach außen verhindert wird.

2. Applikator gemäß Anspruch 1, **gekennzeichnet durch**:
einen Aufsatz (12), der auf die Zinken (18, 19) aufgesteckt ist, zum Dichten zwischen den Zinken (18, 19) und der Innenwand des entsprechenden Nasenlochs.

3. Applikator gemäß Anspruch 2, **dadurch gekennzeichnet dass** der Aufsatz aus einem ersten Dichtkegel (23) und einem zweiten Dichtkegel (24) besteht, wobei der Wortbestandteil "Kegel" lediglich darauf hinweisen soll, dass die Dichtkegel (23, 24) auf der dem Körper (11) abgewandten Seite einen unterschiedlichen Querschnitt als auf der dem Körper (11) zugewandten Seite aufweisen, und die Form des Querschnitts der Dichtkegel (23, 24) vom Abstand vom Körper (11) abhängen kann.

4. Applikator gemäß Anspruch 3, **dadurch gekennzeichnet, dass** die beiden Dichtkegel (23, 24) auf ihrer, dem Körper (11) zugewandten Seite über eine Brücke (29) mechanisch verbunden sind.

5. Applikator gemäß einem der Ansprüche 3 bis 4, **dadurch gekennzeichnet, dass** die Dichtkegel (23, 24) auf ihrer, dem Körper (11) abgewandten Seite eine oder mehrere kranzförmige Dichtlippen (25, 26, 27, 28) aufweisen, die mit der Innenwand des entsprechenden Nasenlochs dicht schließen.

6. Applikator gemäß einem der Ansprüche 3 bis5, **dadurch gekennzeichnet, dass** jeder der beiden Dichtkegel (23, 24) ein inneres Teil (31, 32) und einem äußeren Kegel (33, 34) sowie Rippen (30) umfasst, wobei eine innere Mantelfläche des inneren Teils (31, 32) mit der äußeren Mantelfläche der entsprechenden Zinke (18, 19) eine Passung bildet, und die Rippen das innere Teil (31, 32) mit dem entsprechenden äußeren Kegel (33, 34) so mechanisch verbinden, dass zwischen dem inneren Teil (31, 32) und dem entsprechenden äußeren Kegel (33, 34) Gas parallel zum inneren Teil (31, 32) fließen kann.

7. Applikator gemäß einem der Ansprüche 5 oder 6, **dadurch gekennzeichnet, dass** das innere Teil (31, 32) an seinem, dem Körper (11) zugewandtem Ende über den äußeren Kegel (33, 34) in Richtung Körper (11) hinausragt, wobei das innere Teil (31, 32) auf Höhe des dem Körper zugewandten Endes des äußeren Kegels (33, 34) eine Umfangsstufe aufweist, die eine beilagscheibenförmige Membran (13, 14) gegen das dem Körper (11) zugewandte Ende des entsprechenden äußeren Kegels (33, 34) drückt, wobei die Membran (13, 14) die Öffnung zwischen dem inneren Teil (31, 32) und dem äußeren Kegel (33, 34) verschließt.

8. Applikator gemäß einem der Ansprüche 2 bis 7, **dadurch gekennzeichnet, dass** die Zinken (18, 19) an ihrem nasenseitigen Ende einen Wulst (35, 36) aufweisen, dessen äußerer Durchmesser größer als der lichte Querschnitt des Aufsatzes (12) ist.

9. Applikator gemäß einem der Ansprüche 1 bis 8, **dadurch gekennzeichnet, dass** der Körper (11) eine Öffnung (37) aufweist, in der eine Blende (15) eingelassen ist, wobei die Blende (15) eine Nut (39) aufweist, in die der Rand der Öffnung (37) des Körpers (11) eingreift, wobei die Blende (15) eine Öffnung (38) aufweist, die auf der dem Hohlraum (22) zugewandten Seite durch eine flexible Membran (16, 17) verschlossen ist, so dass Gas aus der Umgebung durch die Öffnung (38) in der Blende (15) in den Hohlraum (22) fließen kann, wobei ein Gasfluss aus dem Hohlraum (22) in die Umgebung durch die Öffnung (38) in der Blende (15) nicht möglich ist.

## Claims

1. Applicator for a nasal cannula, comprising:
a body (11) enclosing a hollow space (22);
a first tube connection (20) and a second tube connection (21) for supplying a respirable gas into the hollow space (22), the first and second tube connections being integrally formed on the body (11);
a first prong (18) and a second prong (19) for administering the respirable gas into the nostrils of a person, the first and second prongs (18, 19) being integrally formed on the body (11);
a valve (15, 16, 17) in a wall of the body (11),
**characterized in that**
the valve is constructed such that respirable gas can flow from the outside through the valve (15, 16, 17, 38, 42) into the hollow space (22) and further to the prongs (18, 19) but on the other hand a significant gas flow from the hollow space (22) through the valve (15, 16, 17, 38, 42) to the outside is prevented.

2. Applicator according to claim 1,
**characterized by**:
a top (12) slipped over the prongs (18, 19) for sealing between the prongs (18, 19) and the inner wall of the respective nostril.

3. Applicator according to claim 2,
**characterized in that**
the top is formed of a first sealing cone (23) and a second sealing cone (24), wherein the word component "cone" is to merely indicate that the sealing cones (23, 24) on the side facing away from the body (11) have a different cross-section than on the side facing the body (11), and the shape of the cross-section of the sealing cones (23, 24) can depend on the distance from the body (11).

4. Applicator according to claim 3,
**characterized in that**
the two sealing cones (23, 24) are mechanically connected by a bridge (29) on their side facing the body (11).

5. Applicator according to any of the claims 3 to 4,
**characterized in that**
on their side facing away from the body (11), the sealing cones (23, 24) are provided with one or more rim-shaped sealing lips (25, 26, 27, 28) that produce a tight seal with the inner wall of the respective nostril.

6. Applicator according to any of the claims 3 to 5,
**characterized in that**
each of the two sealing cones (23, 24) comprises an inner part (31, 32) and an outer cone (33, 34) as well as ribs (30), an inner circumferential surface of the inner part (31, 32) forming a fit with the outer circumferential surface of the respective prong (18, 19) and the ribs mechanically connecting the inner part (31, 32) to the respective outer cone (33, 34) so that a gas can flow parallel to the inner part (31, 32) between the inner part (31, 32) and the respective outer cone (33, 34).

7. Applicator according to any of the claims 5 or 6,
**characterized in that**
the inner part (31, 32) projects on its end facing the body (11) over the outer cone (33, 34) in the direction of the body (11), the inner part (31, 32) being provided with a circumferential step at the height of the end of the outer cone (33, 34) facing the body, which circumferential step presses a shim-shaped membrane (13, 14) against the end of the respective outer cone (33, 34) facing the body (11), the membrane (13, 14) sealing the opening between the inner part (31, 32) and the outer cone (33, 34).

8. Applicator according to any of the claims 2 to 7,
**characterized in that**
the prongs (18, 19) on their nose-side end comprise a collar (35, 36) whose outer diameter is larger than the clear cross-section of the top (12).

9. Applicator according to any of the claims 1 to 8,
**characterized in that**
the body (11) comprises a hole (37) embedding a cover (15), the cover (15) having a groove (39), the edge of the hole (37) of the body (11) engaging into said groove, the cover (15) having an aperture (38) being sealed by a flexible membrane (16, 17) on the side facing the hollow space (22) so that gas can flow from the ambiance through the aperture (38) in the cover (15) into the hollow space (22), a gas flow from the hollow space (22) into the ambiance through the aperture (38) in the cover (15) being impossible.

## Revendications

1. Applicateur pour une canule nasale comprenant :
un corps (11) entourant un espace creux (22) ;
un premier raccord de tuyau (20) et un deuxième raccord de tuyau (21) pour la fourniture d'un gaz respirable dans l'espace creux (22), le premier et le deuxième raccord de tuyau étant formés intégralement sur le corps (11) ;
une première pointe (18) et une deuxième pointe (19) pour l'administration du gaz respirable dans les narines d'une personne, la première et la deuxième pointe (18, 19) étant formées intégralement sur le corps (11) ;
une soupape (15, 16, 17) dans une paroi du corps (11),
**caractérisé en ce que**
la soupape est construite de telle manière qu'un gaz respirable puisse couler de l'extérieur par la soupape (15, 16, 17, 38, 42) dans l'espace creux (22) et jusqu'aux pointes (18, 19), et que, d'autre part, un flux considérable de gaz à partir de l'espace creux (22) par la soupape (15, 16, 17, 38, 42) à l'extérieur soit empêché.

2. Applicateur selon la revendication 1,
**caractérisé par** :
un embout (12) qui est enfiché sur les pointes (18, 19) afin de réaliser une étanchéité entre les pointes (18, 19) et la paroi intérieure de la narine respective.

3. Applicateur selon la revendication 2,
**caractérisé en ce que**
l'embout est composé d'un premier cône d'étanchéité (23) et d'un deuxième cône d'étanchéité (24), l'élément verbal « cône » devant simplement indiquer que les cônes d'étanchéité (23, 24), de leur côté détourné du corps (11), ont une coupe transversale différente de celle de leur côté tourné vers le corps (11), et la forme de la coupe transversale des cônes d'étanchéité (23, 24) pouvant dépendre de la distance par rapport au corps (11).

4. Applicateur selon la revendication 3,
**caractérisé en ce que**
les deux cônes d'étanchéité (23, 24) sont raccordés mécaniquement par un pont (29) de leur côté tourné vers le corps (11).

5. Applicateur selon l'une quelconque des revendications 3 à 4,
**caractérisé en ce que**
les cônes d'étanchéité (23, 24), de leur côté détourné du corps (11), ont une ou plusieurs lèvres d'étanchéité (25, 26, 27, 28) en forme de couronne qui produisent un étanchement avec la paroi intérieure de la narine respective.

6. Applicateur selon l'une quelconque des revendications 3 à 5,
**caractérisé en ce que**
chacun des deux cônes d'étanchéité (23, 24) a une partie intérieure (31, 32) et un cône extérieur (33, 34) ainsi que des nervures (30), une surface d'enveloppe intérieure de la partie intérieure (31, 32) formant un ajustement avec la surface d'enveloppe extérieure de la pointe (18, 19) respective, et les nervures raccordant mécaniquement la partie intérieure (31, 32) au cône extérieur (33, 34) respectif de telle manière que du gaz puisse couler entre la partie intérieure (31, 32) et le cône extérieur (33, 34) respectif parallèlement par rapport à la partie intérieure (31, 32).

7. Applicateur selon l'une quelconque des revendications 5 ou 6,
**caractérisé en ce que**
la partie intérieure (31, 32), à son extrémité tournée vers le corps (11), dépasse au-delà du cône extérieur (33, 34) dans la direction du corps (11), la partie intérieure (31, 21), au niveau de l'extrémité du cône extérieur (33, 34) tournée vers le corps, ayant un gradin circonférentiel qui presse une membrane (13, 14) en forme de cale contre l'extrémité du cône extérieur (33, 34) respectif tournée vers le corps (11), la membrane (13, 14) fermant l'ouverture entre la partie intérieure (31, 32) et le cône extérieur (33, 34).

8. Applicateur selon l'une quelconque des revendications 2 à 7,
**caractérisé en ce qu'**
à leur extrémité de côte de nez, les pointes (18, 19) ont un bourrelet (35, 36) dont le diamètre extérieur est supérieur à la coupe transversale intérieure de l'embout (12).

9. Applicateur selon l'une quelconque des revendications 1 à 8,
**caractérisé en ce que**
le corps (11) a une ouverture (37) dans laquelle un couvercle (15) est inséré, le couvercle (15) ayant une rainure (39) dans laquelle le bord de l'ouverture (37) du corps (11) s'engrène, le couvercle (15) ayant une ouverture (38) qui est fermée du côte tourné vers l'espace creux (22) par une membrane flexible (16, 17) de telle manière que du gaz puisse couler de l'environnement par l'ouverture (38) du couvercle (15) dans l'espace creux (22), un flux de gaz à partir de l'espace creux (22) dans l'environnement par l'ouverture (38) du couvercle (15) étant impossible.
